(19) Europäisches Patentamt
European Patent Office
Office européen des brevets

(11) Publication number: **0 132 283 A2**

(12) **EUROPEAN PATENT APPLICATION**

(21) Application number: **84303932.2**

(22) Date of filing: **11.06.84**

(51) Int. Cl.⁴: **C 07 D 249/08**, A 01 N 43/653

(30) Priority: **25.07.83 GB 8319984**

(43) Date of publication of application: **30.01.85 Bulletin 85/5**

(84) Designated Contracting States: **BE CH DE FR GB IT LI NL SE**

(71) Applicant: **IMPERIAL CHEMICAL INDUSTRIES PLC, Imperial Chemical House Millbank, London SW1P 3JF (GB)**

(72) Inventor: **Sugavanam, Balasubramanyan, 11 Kiln Ride, Wokingham Berkshire (GB)**

(74) Representative: **Alner, Henry Giveen Hamilton et al, Imperial Chemical Industries PLC Legal Department: Patents P.O. Box 6 6, Bessemer Road, Welwyn Garden City Herts, AL7 1HD (GB)**

(54) **Optically active fungicidal diaryltriazolylethanol derivative.**

(57) The minus enantiomer of the compound of formula:

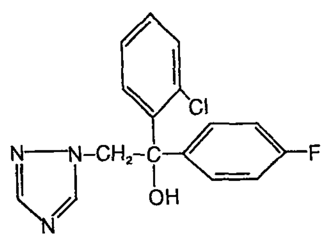

and esters, ethers, salts and metal complexes thereof, are useful as plant fungicides.

EP 0 132 283 A2

ACTORUM AG

TITLE MODIFIED see front page

# OPTICALLY ACTIVE FUNGICIDAL COMPOUND

This invention relates to an optical isomer of a certain triazole derivative, to a process for obtaining this isomer, to fungicidal compositions containing it, and to a method of combating fungal infection in plants using it. The invention is related to that of European Patent Specification No 15756, the disclosure of which Application is incorporated herein by reference.

The triazole derivative has the formula:

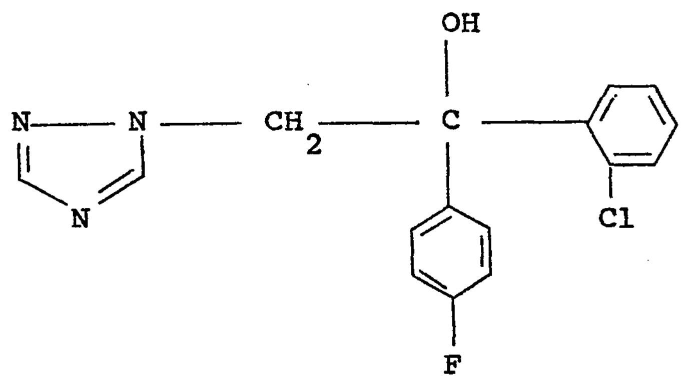

It will be appreciated that this compound contains one chiral centre. This means that the compound can exist in two optical isomeric forms. These isomers are enantiomers (ie. mirror images) of each other.

We have now found that when the two enantiomers are separated there is a significant separation of the fungicidal activity between the two enantiomers. Thus we have found that one of them, the (-) enantiomer has higher fungicidal activity than the racemate or the other isomer, ie. the (+) enantiomer.

The invention therefore provides the minus enantiomer of the compound having the formula:

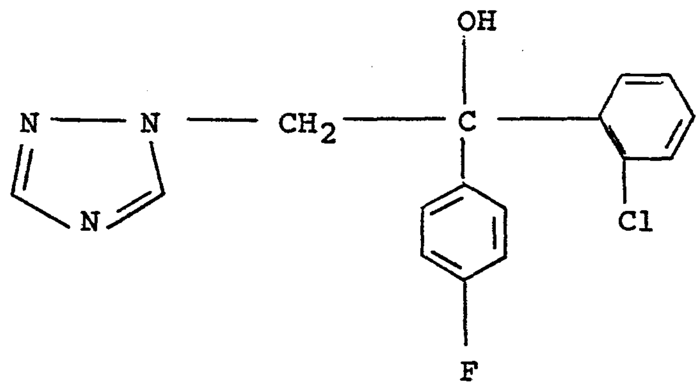

and esters, ethers, acid addition salts and metal complexes of this enantiomer. The optical purity of an enantiomer can be established by nuclear magnetic resonance techniques using a chiral shift reagent.

Suitable salts are salts with inorganic or organic acids, eg. hydrochloric, nitric, sulphuric, toluenesulphonic, acetic or oxalic acid. The esters are suitably alkanoates (eg. acetates) and the ethers are suitably alkyl (eg. methyl or ethyl), aryl (eg. phenyl) or aralkyl (eg. benzyl) ethers.

The metal complex is suitably one including copper, zinc, manganese or iron. It preferably has the general formula:

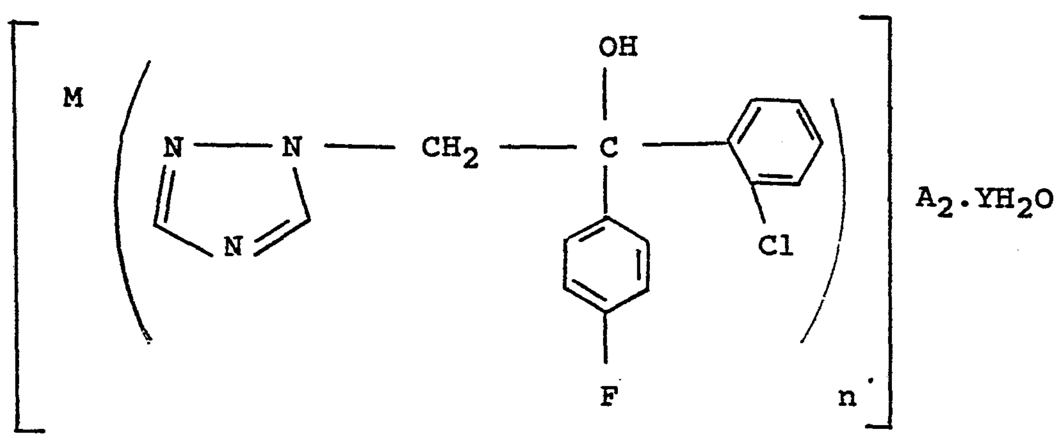

wherein M is a metal, A is a anion (eg. a chloride, bromide, iodide, nitrate, sulphate or phosphate anion), n is 2 or 4, and Y is 0 or an integer of 1 to 12.

The racemate of the compound of formula (I), or a salt thereof, can be prepared as described in European Patent Specification No 15756.

The salts, metal complexes, ethers and esters of the compounds of formula (I) can be prepared from the latter in known manner. For example, the complexes can be made by reacting the uncomplexed compound with a metal salt in a suitable solvent.

The resolution of the racemate of formula I into the constituent enantiomers can be performed by known methods. Examples of these methods are (1) forming the diastereoisomeric salts or esters of the compound of general formula (I) with an optically active acid (eg. camphor sulphonic acid), separating the isomeric salts or esters and converting the separated isomeric salts or esters into the enantiomers of the compound of general formula (I); (2) forming the diastereoisomeric carbamates of the compound of general formula (I) by reacting a haloformate (eg. chloroformate) of the latter with an optically active amine (eg. α-methylbenzylamine), separating the isomeric carbamates, and converting the separated isomeric carbamates into the enantiomers of the general formula (I); or (3) resolving the mixtures using enantioselective crystallisation techniques (Leigh, Chemistry and Industry, 1970, pages 1016-1017, and _ibid_, 1977, page 36). The separation of the diastereoisomeric salts, esters and carbamates can be achieved by for example crystallisation techniques or by high pressure liquid chromatography (HPLC). A schematic representation of two schemes is set below.

(I)

Racemate

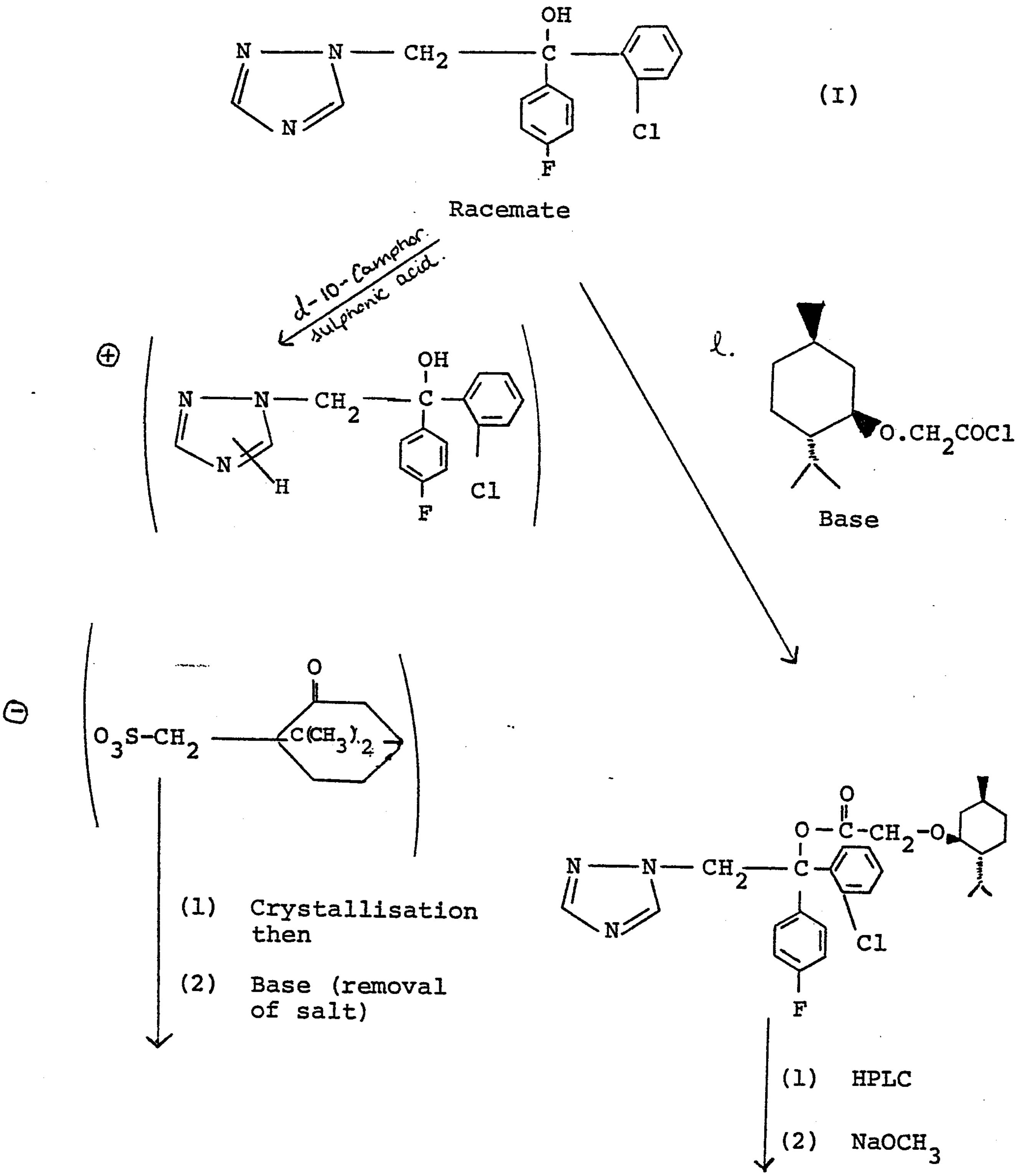


+ and - enantiomers of racemate (I)

SCHEME 1

+ and - enantiomers of racemate (I)

SCHEME 2

The minus enantiomer of the invention and its ethers, esters, salts and metal complexes (hereinafter referred to as "the compounds") have fungicidal activity particularly against the diseases:

Piricularia oryzae on rice
Puccinia recondita, Puccinia striiformis and other rusts on wheat, Puccinia hordei, Puccinia striiformis and other rusts on barley, and rusts on other hosts eg. coffee, apples, vegetables and ornamental plants
Plasmopara viticola on vines
Erysiphe graminis (powdery mildew) on barley and wheat and other powdery mildews on various hosts such as Sphaerotheca fuliginea on cucurbits (eg. cucumber) Podosphaera leucotricha on apples and Uncinular necator on vines
Helminthosporium spp. on cereals
Cercospora arachidicola on peanuts and other Cercospora species on for example sugar beet, bananas and soya beans
Botrytis cinerea (grey mould) on tomatoes, strawberries, vines and other hosts
Venturia inaequalis (scab) on apples

Activity has been demonstrated against a broad range of fungi in vitro. They have activity against various post-harvest diseases on fruit (eg. Penicillium digatatum and italicum on oranges and Gloeosporium musarum on bananas). Further some of the compounds are active as seed dressings against : Fusarium spp., Septoria spp., Tilletia spp. (ie. bunt, a seed borne disease of wheat), Ustilago spp., Helminthosporium spp. on cereals, Rhizoctonia solani on cotton and Corticium sasakii on rice.

The minus enantiomer may be used as such for fungicidal purposes but are more conveniently formulated into compositions for such usage. The invention thus provides also a fungicidal composition comprising the minus enantiomer as hereinbefore defined, and a carrier or diluent.

The invention also provides a method of combating fungal diseases in a plant, which method comprises applying to the plant, to seed of the plant or to the locus of the plant or seed the minus enantiomer as hereinbefore defined.

The minus enantiomer can be applied in a number of ways, for example it can be formulated or unformulated, directly to the foliage of a plant, to seeds or to other medium in which plants are growing or are to be planted, or it can be sprayed on, dusted on or applied as a cream or paste formlation, or it can be applied as a vapour. Application can be to any part of the plant, bush or tree, for example to the foliage, stems, branches or roots, or to soil surrounding the roots, or the the seed before it is planted.

The term "plant" as used herein includes seedlings, bushes and trees. Furthermore, the fungicidal method of the invention includes preventative, protectant, proplylactic and eradicant treatment.

The minus enantiomer is preferably used for agricultural and horticultural purposes in the form of a composition.

The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be

preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methyl-pyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The minus enantiomer can be mixed in the dry state with a pyrotechnic mixture to form a composition suitable for generating in enclosed spaces a smoke containing the compounds.

Alternatively, the minus enantiomer may be used in a microencapsulated form.

By including suitable additives, for example additives for improving the distribution, adhesive power and resistance to rain on treated surfaces, the different compositions can be better adapted for various utilities.

The minus enantiomer can be used as mixtures with fertilisers (eg. nitrogen-, potassium- or phosphorus-containing fertilisers). Compositions comprising only granules of fertiliser incorporating, for example coated with, the compound, are preferred. Such granules suitably contain up to 25% by weight of the minus enantiomer. The invention therefore also provides a fertiliser composition comprising a minus enantiomer as hereinbefore defined.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium layryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenyl and octylcresol. Other non-ionic agents are the partial esters derived from long chain fatty acids and hexitol anhydrides, the condensation products of

the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s) (ie. minus enantiomer), the concentrate to be diluted with water before use. These concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-58%, for example 25-60%, by weight of the active ingredient(s). These concentrates suitably contain organic acids (eg. alkaryl or aryl sulphonic acids such as xylenesulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containg 0.0005% or 0.01% to 10% by weight of active ingredient(s) may be used.

The compositions of this invention can comprise also other compound(s) having biological activity eg. compounds having similar or complementary fungicidal or plant growth regulating activity or compounds having herbicidal or insecticidal activity.

The other fungicidal compound can be for example one which is capable of combating ear diseases of cereals (eg. wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, dimethirimol, bupirimate, chlorothalonil, vinclozolin, procymidone, iprodione, metalaxyl, forsetyl-aluminium, methfuroxan, nitrotal-isopropyl, triadimefon, thiabendazole, etridiazole, triadimenol, biloxazol, dithianon, binapacryl, quinomethionate, guazitine, dodine, fentin acetate, fentin hydroxide, dinocap, folpet, dichlofluanid, ditalimphos, kitazin, cycloheximide, dichlobutrazol, a dithiocarbamate, a copper compound, a mecury compound, 1-(2-cyano-2-methoxyiminoacetyl)-3-ethyl urea, fenapanil, ofurace, propiconazole, etaconazole and fenpropemorph.

The minus enantiomer of the invention can be mixed with soil, peat or other rooting media for the protection of plants against seed-borne, soil-borne or foliar fungal diseases.

Suitable insecticides are pirimor, croneton, dimethoate, metasystox and formothion.

The plant growth regulating compound can be one which controls weeds or seedhead formation, improves the level of the compounds of general formula (I), selectively controls the growth of the less desirable plants (eg. grasses) or causes the compound of general formula (I) to act faster or slower as a plant growth regulating agent. Examples of suitable agents are the gibberellins (eg. $GA_3$, $GA_4$ or

$GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acids (eg. triiodobenzoic acid), morphactins (eg. chlorfluorecol), maleic hydrazide, glyphosate, glyphosine, long chain fatty alcohols and acids, dikegulac, fluoridamid, mefluidide, substituted quaternary ammonium and phosphonium compounds (eg. chlormequat or chlorphonium), ethepon, carbetamide, methyl-3,6-dichloranisate, daminozide, asulam, abscissic acid, isopyrimol, 1(4-chlorophenyl)-4,6-dimethyl-2-oxo-1,2-dihydropyridine-3-carboxylic acid, hydroxybenzonitriles (eg. bromoxynil), difenzoquat, benzoylprop-ethyl 3,6-dichloropicolinic acid.

The following Examples illustrate the invention; the temperatures are given in degrees centigrade (°).

EXAMPLE 1

This Example illustrates the preparation of - and + forms from (±) 1-(4-fluorophenyl)-1-(2-chlorophenyl)-2-(1,2,4-triazol-1-yl)-ethan-1-ol having the formula:

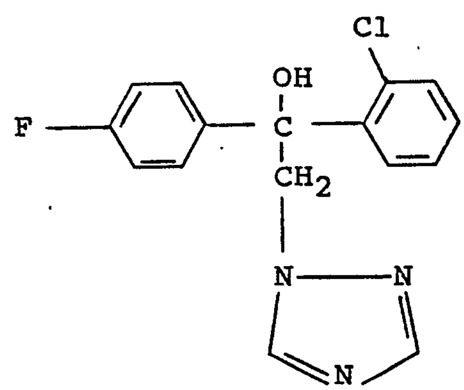

A mixture of (±) 1-(4-fluorophenyl)-1-(2-chlorophenyl)-2-(1,2,4-triazol-1-yl)-ethan-1-ol (20g) and d-10-camphor sulphonic acid (18g) in acetonitrile (500ml) was refluxed on a steam bath for 4 hours and left overnight at room temperature. The needle shaped crystals that were formed was filtered off and the mother liquor was retained.

The filtered solid (14g) was crystallised twice with acetonitrile, m.p. 165-168°C $[\alpha]_D^{20}$-82°, $CHCl_3$, C2.5. The (-)-1(4-fluorophenyl)-1(2-chlorophenyl)-2-(1,2,4-triazol-1-yl)-ethan-1-ol was obtained by neutralising the salt with $NaHCO_3$ solution. The free base was crystallised from ethyl acetate-petrol (40-60°) m.p. 127-130°, $[\alpha]_D^{20}$-67, $CHCl_3$; C, 2.5. The mother liquor from the retained filtrate (as above) was removed to dryness <u>in vacuo</u> and the residual gum was digested with ether to produce an amorphous solid. This was filtered off and washed with ether a number of times. The salt was recrystallised twice with ethyl acetate-ether mixture, $[\alpha]_D^{20}$ +120°, $CH_3Cl_3$, C, 2.5 m.p. 150-170°. The (-) isomer of the title compound was obtained by neutralising the salt with $NaHCO_3$ solution and recrystallising from ethylacetate- petrol (40-60°), m.p. 127-130°, $[\alpha]_D^{20}$ + 61.5, $CHCl_3$, C, 2.5.

EXAMPLE 2

This Example shows that the enantiomer of the compound of formula I has a higher fungicidal activity than the racemate and the + enantiomer.

Each compound was applied in a powder formulation to 25g batches of seeds at 5, 10, 25 and 50ppm. Treated seeds were sown, 15 per 3 inch pot, in John Innes Number2 potting compost and pots were watered uniformly overhead. Four replicates per treatment were used and these were distributed in a randomised block design.

For the tests with <u>Erysiphe graminis tritici</u>, wheat cv. Flinor was used and the plants were grown in a glasshouse with a temperature range 19°-27°C. <u>Erysiphe graminis hordei</u> tests utilized Maris Otter barley grown under similar conditions. The same cultivar of barley was used for tests against <u>Rhynchosporium secalis</u> but plants were grown at 21°C in a growth room with an 8hour daylength. Finally, for the tests against <u>Puccinia</u>

*recondita* wheat cv. Armada was grown in a growth room at 20°C with an 8 hour daylength.

In the cereal mildew tests, plants were inoculated 7 days after sowing by shaking heavily infected stock plants overhead. Disease was assessed 7 days after inoculation. Inoculations with *Rhynchosporium secalis* were achieved using a 0.05% Tween spore suspension at 200,000 spores per ml 7 days after sowing followed by an incubation period of 24 hours at 20°C in a humid room; plants were assessed 9 days after inoculation. The rust inoculations were made 7 days after sowing and a 0.05% Tween spore suspension at 200,000 spores per ml was sprayed onto the plants; incubation followed in a humid room at 20°C for 24 hours prior to assessment 11 days after inoculation.

For each replicate, ten leaves were assessed for disease and the mean percentage disease for the 4 replicates was calculated. Disease grades were assigned as follows:

0 = 60-100% of disease on untreated plants
1 = 26-59% of disease on untreated plants
2 = 6-25% of disease on untreated plants
3 = trace - 5% of disease on untreated plants
4 = no disease

The results obtained in side-by-side tests with the racemate, its + enantiomer and its - enantiomer are presented in Table I. The values given are the rates in ppm at which each isomer gave a grade 3(trace - 5% disease control) in each test.

TABLE I

| | Erysiphe graminis tritici | Erysiphe graminis hordie | Puccinia recondita | Rhynchosporium secalis |
|---|---|---|---|---|
| Racemate | 5-10 | 5-10 | 50 | 50 |
| +Enantiomer | 50 | 25 | >50 | >50 |
| -Enantiomer | <5 | 5 | 25 | 25 |

'>' means "greater than".
'<' means "less than".

HGHA/jlw
PP 32816
30 May 1984

1. The minus enantiomer of the compound formula :

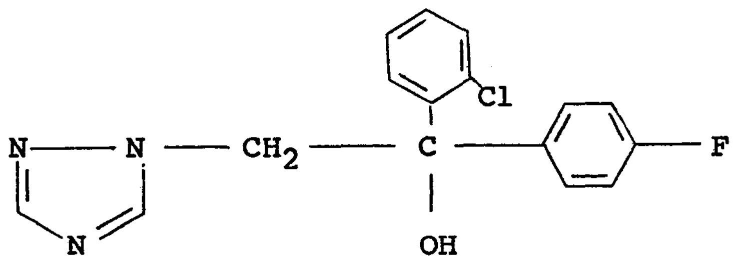

and esters, ethers, salts and metal complexes thereof.

2. A process for making the enantiomeric claimed in claim 1 which comprises forming the diastereoisomeric salts or esters of the compound of general formula (I) :

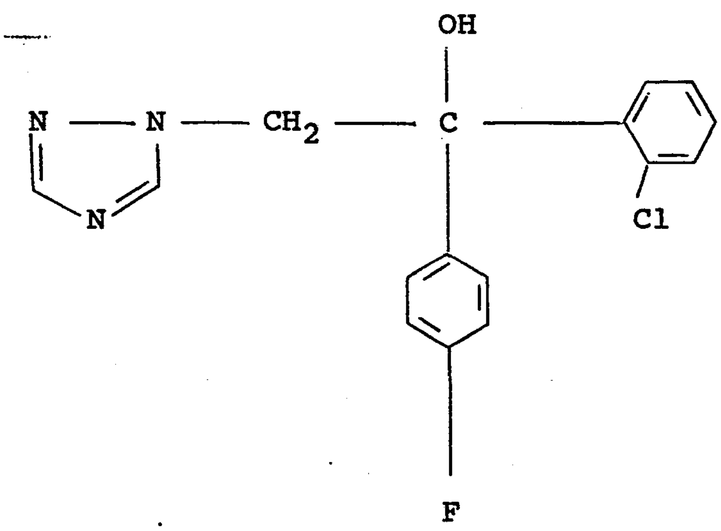

(I)

Racemate

with an optically active acid such as camphor sulphonic acid, separating the isomeric salts or esters and converting the separated isomeric salts or esters into the enantiomers of the compound of general formula I.

3. A process for making the enantiomer claimed in claim 1 which comprises forming the diastereocarbamates of the compound of formula :

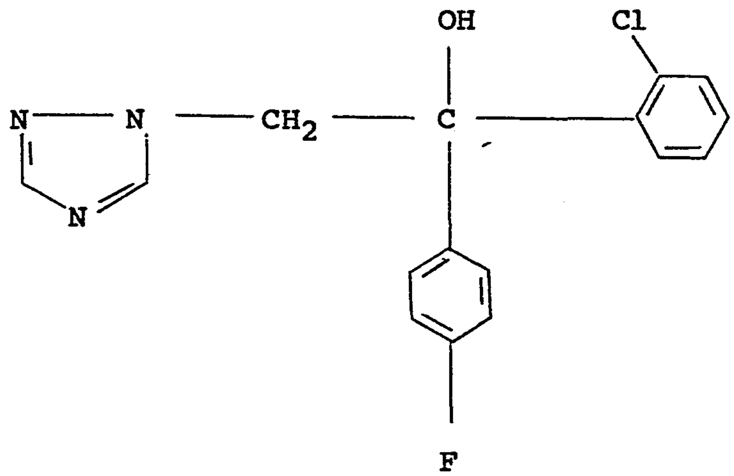

by forming a haloformate (eg. chloroformate) of the compound and bringing this into reaction with an optically active amine such as α-methylbenzylamine; separating the isomeric carbamates and converting these into the enantiomers of the compound and retaining the minus enantiomer.

4. A process as claimed in claim 2 or claim 3 wherein the diastereoisomeric esters or carbamates are separated by an enantioselective crystallisation technique or by the use of high pressure liquid chromatography.

5. A fungicidal composition comprising, as an active ingredient, the minus enantiomeric claimed in claim 1 together with a carrier therefor.

6. A process for combating fungal diseases in a plant which comprises applying to the plant, to the seed of the plant, or to the locus of the plant or seed, the minus enantiomer claimed in claim 1, or a composition as claimed in claim 5.

7. The processes of Scheme 1 and Scheme 2 as hereinbefore set forth.

8. The process as described for preparing the minus enantiomer claimed in claim 1 with particular reference to Example 1.